# EUROPÄISCHE PATENTSCHRIFT

(11) **EP 0 894 450 B1**
(45) Veröffentlichungstag und Bekanntmachung des Hinweises auf die Patenterteilung: **07.05.2003**
(21) Anmeldenummer: 98114393.6
(22) Anmeldetag: 31.07.1998
(51) Int. Cl.: A45D 24/30

(54) **Reinigungskamm mit Zähnen, die in ihrer peripheren Oberfläche eine Rauhigkeit aufweisen, und Methode zur Herstellung eines Reinigungskamms mit hoher mechanischer Festigkeit**
Cleaning comb with teeth having a predetermined roughness on their outer surface and method of manufacturing a cleaning comb of high mechanical strength
Peigne de nettoyage à dents munis d'une surface rugueuse et procédé de production d'un peigne de nettoyage à résistance méchanique elevée

(30) Priorität: 01.08.1997 US 904762
(43) Veröffentlichungstag der Anmeldung: 03.02.1999
(73) Patentinhaber: Assistance S.R.L., 1001 Buenos Aires (AR)
(72) Erfinder: Sanz, Juan Martin, Buenos Aires (AR)
(74) Vertreter: Gritschneder, Martin, Dipl.-Phys.

(56) Entgegenhaltungen:
- DE-C- 339 538
- DE-C- 634 125
- GB-A- 604 963
- US-A- 2 546 541
- US-A- 2 605 773
- US-A- 4 671 303
- US-A- 5 636 646

## Beschreibung

Die vorliegende Erfindung betrifft einen Reinigungskamm mit Zähnen, die in ihrer peripheren Oberfläche eine Rauhigkeit aufweisen, und eine Methode zur Herstellung eines Reinigungskamms mit hoher mechanischer Festigkeit.

Obwohl vom Stand der Technik verschiedene Arten von Kämmen zur Reinigung von fest an den Haaren anhaftenden Teilchen, insbesondere Kämme für Läuse, bekannt sind, ist das Problem der Entfernung fremder an den Haaren von Menschen oder Tieren anhaftender Stoffe noch nicht gelöst. Das größte Problem von allen ist das der Nissen (daß heißt der Eier, der sich in menschlichen Haaren einnistenden Läuse), weil diese sich am festesten an den Haaren anhaften.

In der Patentschrift USA N° 4 612 945 wird ein Kamm zur Reinigung der Haare von Läusen und Nissen beschrieben: Der Kamm weist Zähne mit einem dreiecksförmigen Querschnitt auf. Der Durchmesser der Zähne nimmt von den Befestigungsenden zu den Anwendungsenden hin derart ab, daß der Abstand zwischen benachbarten Zähnen in der Nähe der Anwendungsenden größer ist als in der Nähe des Griffs. Die Zähne sind derart angeordnet, daß ihre Längsachsen parallel sind und, daß sie sich in zwei parallelen Ebenen befinden, wobei benachbarte Zähne abwechselnd zu einer der beiden Ebenen gehören. Mit dieser Anordnung wird ein Schereneffekt erreicht um die Läuse und Nissen aus den Haaren zu entfernen. Dieser Kamm hat jedoch einige Nachteile. Die Zähne haben eine relativ geringe Länge wodurch nicht in jedem Haartyp gewährleistet ist mit den Zahnspitzen bis zur Kopfhaut zu gelangen. Die Abstände zwischen den Spitzen der Anwendungsenden der Zähne sind ziemlich groß, so daß die Wirksamkeit gegen die Läuse und insbesondere gegen die Nissen erheblich vermindert ist. Außerdem weisen die Zähne durch ihre Dreiecksform scharfe Ränder auf, und haben daher die Neigung zu dem absolut unerwünschten Effekt die Oberfläche der Haare zu verletzen.

In der Patentschrift USA 4 671 303 werden ein Nissenkamm und eine Methode zur Herstellung desselben beschrieben. Der Kamm umfaßt eine Vielzahl von in einem Griff befestigten aus Metall hergestellten Zähnen, mit einem Abstand zwischen den Zähnen von ca. 100 µm bis 120 µm. Jeder Zahn ist in der Nähe seines Befestigungsendes mit einem länglichen Kanal versehen, der einem jeweiligen länglichen Verbindungselement im Griff komplementär entspricht, wodurch sichergestellt wird daß die Zähne sowohl während des Herstellung des Kammes als auch während seines Gebrauchs parallel gehalten werden. Die Zähne haben eine Nutzlänge, daß heißt einen Teil der nach außen aus dem Griff herausragt und zum Kämmen der Haare verwendet werden kann, von 9 mm oder weniger, und die Spitzen der Zähne sind hakenförmig ausgestaltet um besser die Läuse und die Nissen aus den Haaren entfernen zu können. In vielen Fällen ist die geringe Nutzlänge der Zähne nicht ausreichend um mit dem Kamm bis zur Oberfläche der Kopfhaut zu gelangen. Trotz der spezifischen Form der Zahnspitzen, hat dieser Kamm nicht die gewünschte Wirksamkeit für die Entfernung fest an den Haaren anhaftender Teilchen, wie es zum Beispiel die Nissen sind, da der Abstand zwischen den einzelnen Zähnen viel zu groß ist und die Oberfläche der Zähne glatt ist, wodurch die Mehrzahl der Nissen nicht am Kamm hängenbleibt.

Eine Aufgabe der vorliegenden Erfindung ist es einen Kamm zur Verfügung zu stellen mit wesentlich verbesserten Eigenschaften für die Reinigung jedes beliebigen Haartyps oder ähnlichem, von fest anhaftenden Teilchen und insbesondere von Objekten wie Nissen, die im allgemeinen sehr fest an den Haaren angehaftet sind, ohne gleichzeitig die Haare zu beschädigen.

Eine weitere Aufgabe der vorliegenden Erfindung ist es verschiedene Methoden zur Herstellung von Reinigungskämmen zu liefern, die, trotz der Tatsache daß sie Zähne mit einer relativ großer Nutzlänge haben, eine hohe mechanische Festigkeit gegen Verformungen aufweisen.

Eine weitere Aufgabe der vorliegenden Erfindung ist es Zähne für Kämme herzustellen, die eine periphere Oberfläche mit einer Rauhigkeit aufweisen, die insbesondere spezielle Eigenschaften für die Entfernung von fest an die Haare anhaftenden Teilchen hat.

Diese Aufgabe wird durch die Merkmale der Ansprüche 1 und 16 gelöst. Bevorzugte Gestaltungen der Erfindung sind Gegenstand der Unteransprüche.

In einer bevorzugten Ausführungsform umfaßt der Reinigungskamm Zähne, die auf ihrer peripheren Oberfläche eine Rauhigkeit aufweisen, die parallel in einer Ebene angeordnet sind und einen Abstand untereinander von weniger als 100 µm aufweisen, um in effizienter Art und Weise alle fremden Elemente und kleineren Teilchen, die an den Haaren anhaften, zu entfernen.

Die Zähne haben eine Nutzlänge, daß heißt einen aus dem Griff herausragenden Teil, von mindestens 35 mm um wirksam zu sein, da kürzere Zähne dazu führen, daß die Reinigung von Haaren viel zu arbeitsaufwendig ist, vor allem bei dichtem und reichlichem Haar.

Die Zähne haben einen Querschnitt ohne scharfe Kanten, daher ist der Querschnitt kreisförmig oder oval, und bevorzugt müssen die Zähne eine im wesentlichen zylindrische Form haben damit sie die zu kämmenden und zu reinigenden Haare nicht beschädigen.

Die Zähne werden aus einem Material hergestellt mit einer mechanischen Festigkeit, die gleich oder größer als die von Stahl ist, und bevorzugt werden sie aus rostfreiem Stahl hergestellt mit einem Durchmesser von mindestens 1 mm, um dem Kamm, trotz der relativ großen Nutzlänge, eine hohe mechanische Festigkeit zu verleihen; damit mit dem Kamm beim Reinigen der Haare der von den Nissen geleistete Widerstand überwunden wird.

Als wesentliche Neuheit haben die Zähne des erfindungsgemäßen Kamms eine periphere Oberfläche mit einer feinen Rauhigkeit, die den Effekt hat fremde Teilchen, die an den Haaren anhaften, zu entfernen, und zwar mit einem wesentlich höheren Wirkungsgrad als es von den Zähnen aus dem Stand der Technik her bekannt ist; mit dem Reinigungskamm der vorliegenden Erfindung können, zum Beispiel, zwischen 30 und 50 % mehr Nissen entfernt werden, als es mit aus dem Stand der Technik bekannten Kämmen möglich ist.

Obwohl jede beliebige Rauhigkeit verwendet werden kann, um den Reinigungseffekt des Kammes zu verbessern, werden als bevorzugten Rauhigkeiten mit den besten Reiningungsergebnissen und/oder den geringsten Herstellungskosten, diejenigen verwendet, die aus feinen, die Nissen abschleifenden, nicht die Haare beschädigenden Rillen bestehen. Die Rillen können vorgesehen sein als schraubenförmige Rille mit einem nicht zu großen Gewindegang, oder als parallele ringförmige ringförmige Rillen mit einem genau definierten, nicht zu großen Abstand untereinander.

Bevorzugt werden die Zähne an einem Griff aus aseptischem Material befestigt, wie zum Beispiel rostfreier Stahl, der jeden Tag in heißem Wasser gekocht werden kann ohne sich zu verformen.

Die Rauhigkeit wird in einer weiteren bevorzugten Ausführungsform mit Hilfe einer Methode erzeugt, die die Schritte enthält: ein Schneidwerkzeug auf einer Werkzeugträgervorrichtung anzubringen, wobei die Vorrichtung eine Feder aufweist, um einen gleichförmigen Druck auf einen Richtrollengang in einer Drahtricht- und schneidemaschine auszuüben; einen Zahn der Vielzahl von Zähnen in die Drahtrichtmaschine einzubringen; den besagten Richtrollengang gemeinsam mit besagtem Schneidegerät um den Zahn zu drehen, wobei aus diesem ein Span in Form einer Rille herausgetrennt wird; und gleichzeitig den Zahnes durch den Richtrollengang vorwärtszubewegen, wodurch schließlich die Rille eine Schraubenform in der peripheren Oberfläche des Zahnes bildet; und die Schritte, ausgehend von dem Einbringen eines Zahnes in die Drahtrichtmaschine, für jeden der Vielzahl von Zähnen zu wiederholen.

In einer weiteren bevorzugten Ausführungsform wird die Rauhigkeit erzeugt mit Hilfe der Schritte: einen Zahn in eine Schleifmaschine einzubringen; und parallele ringförmige Rillen in die periphere Oberfläche dieses Zahnes zu schneiden.

Um dem Kamm ein für den Verbraucher angenehmeres Anwendungsende zu verleihen, können die Zähne, während des Positionierens derselben, leicht gegeneinander versetzt angeordnet werden, so daß die Reinigungsenden der Zähne des Kammes einen gekrümmten Rand bilden. In einer weiteren der bevorzugten Ausführungsformen werden die Zähne mit Hilfe eines Lasers auf die erste Schale geschweißt. Der Laserstrahl kann von der Außenseite der Schale her angewandt werden, oder er kann von der Seite der Zähne angewandt werden, in letzterem Falle jedoch nur zwischen zwei jeweils benachbarten Zähnen, um nicht während der Anwendung des Lasers die Struktur der Zähne zu verändern. In diesen Ausführungsformen wird der Griff dadurch verschlossen, daß die zweite Schale mit Hilfe des Lasers auf die erste Schale geschweißt wird.

In einer weiteren bevorzugten Ausführungsform der vorliegenden Erfindung werden die Zähne mit Zinn aufgeschweißt, wobei die Schweißung von der Seite der Zähne her angewandt wird. In diesem Fall wird der Griff, nach Einfügen eines Zwischenstücks aus Plastik zwischen der ersten und der zweiten Schale, mit Hilfe eines Anpreßdrucks verschlossen, wobei das Zwischenstück aus Plastik den Zweck hat den Anpreßdruck zu tragen.

Die vorgenannten Aufgaben werden mit dem Kamm der vorliegenden Erfindung erfüllt, welcher in den nachfolgenden Abbildungen dargestellt wird, und es zeigen:
- die Figur 1A:: eine Aufsicht auf den erfindungsgemäßen Kamm von der Seite der ersten Schale;
- die Figur 1B:: eine Teilseitenansicht des Reinigungskamms in einem Schnitt längs der Linie I-I der Figur 1A;
- die Figur 2A:: eine Seitenansicht eines Zahns mit einer Rauhigkeit in Form einer schraubenförmigen Rille;
- die Figur 2B:: eine Seitenansicht eines Zahns mit einer Rauhigkeit in Form paralleler ringförmiger Rillen;
- die Figur 2C:: eine vergrößerte Sicht des Ausschnitts II der Figur 2A; und
- die Figur 2D:: eine vergrößerte Sicht des Ausschnitts III der Figur 2A.

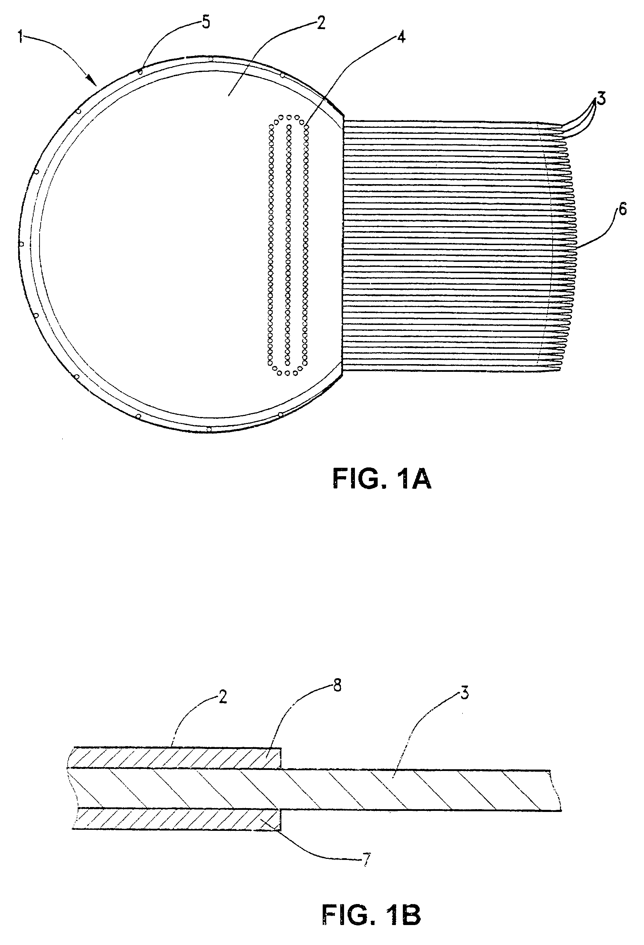

In der Figur 1A ist ein erfindungsgemäßer Kamm 1 dargestellt, der eine Vielzahl von Zähnen oder Stiften 3 umfaßt, und zwei Schalen oder Kappen 7, 8, eine erste Schale 7 und eine zweite Schale 8 (Fig. 1B), die den Griff 2 bilden. Die Anzahl der Zähne 3 kann zwischen 10 und 100 betragen, und bevorzugt umfaßt der Kamm 1 33 einzelne Zähne 3.

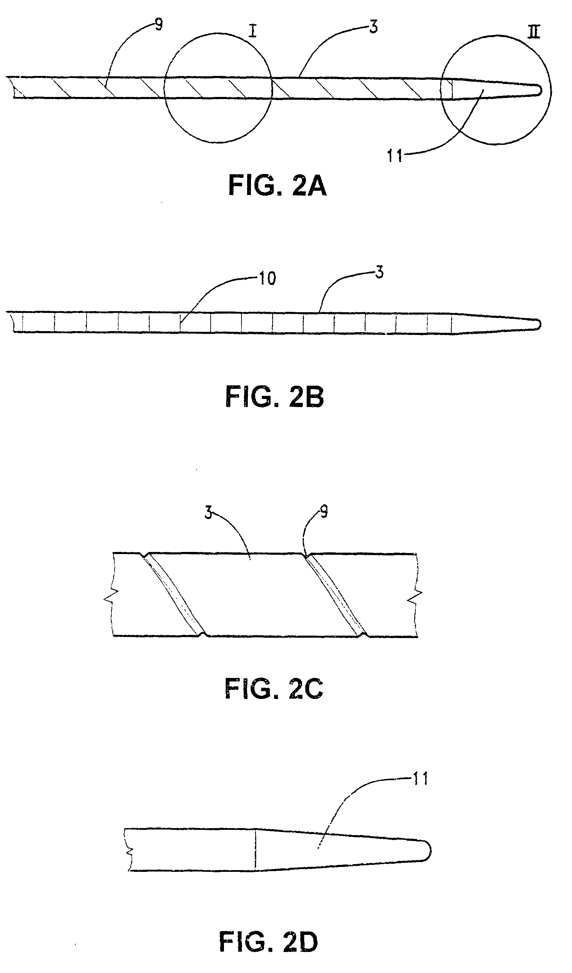

Die Zähne 3 bestehen aus zwei Teilen. Ein aktiver oder nützlicher Teil, der an seinem Anwendungsende in einer konischen abgerundeten Spitze 11 endet (Figuren 2A und 2D) und der einen im wesentlichen zylindrischen Körper hat, welcher seinerseits eine mechanisch bearbeiteten Oberfläche mit einer schraubenförmigen Rille 9 (Figuren 2A und 2C) oder mehreren ringförmigen (Figur 2B) Rillen 10 aufweist. Der andere Teil jedes Zahns 3 ist der Befestigungsteil der in den Griff 2 ragt und dort an der ersten Schale 7 befestigt ist.

Die Zähne 3 werden aus geraden, zylindrischen Elementen gebildet mit einem Durchmesser zwischen 1,0 und 1,5 mm, und bevorzugt mit einem Durchmesser von ca. 1,25 mm mit einer Toleranz von ca. 0,01 mm, und einer Toleranz der Geradheit des zylindrischen Elements von 0,02 mm (Figuren 2A und 2B), wodurch die Zähne 3 mit einem solchen Durchmesser dem Kamm 1 die hohe mechanische Festigkeit verleihen, die wegen der großen Nutzlänge der Zähne 3 benötigt wird.

Die Zähne 3 weisen in ihrer peripheren Oberfläche eine Rauhigkeit auf, die bevorzugt aus einer schraubenförmigen Rille 9 (Figur 2A) oder aus mehreren parallelen ringförmigen ringförmigen Rillen 10 (Figur 2B) besteht.

Zur Herstellung eines Zahnes wird eine handelsübliche Drahtricht- und schneidemaschine verwendet, in der an einem der Richtrollengänge ein Schneidwerkzeug auf einer Werkzeugträgervorrichtung mit einer Feder für die Ausübung eines konstanten Druckes angebracht wird.

Dieses Schneidwerkzeug dreht sich gemeinsam mit dem Richtrollengang um das gerade, zylindrische Element und trennt aus diesem einen Span in Form einer Rille 9. Durch das Vorwärtsbewegen des geraden, zylindrischen Elements durch den Richtrollengang, erhält die Rille 9 schließlich die Form einer Schraube 9 in der peripheren Oberfläche des Zahnes 3. Der Gewindegang der schraubenförmigen Rille 9 (Figur 2A) ist kleiner als 4 mm und bevorzugt kleiner als 2 mm.

Bis hierher bleibt das gerade Element mit seiner mit einer schraubenförmigen Rille versehenen Oberfläche in seiner zylindrischen Form erhalten. Dieses Element wird nun in eine spitzenlose Schleifmaschine eingebracht, wo die Spitze 11 mit einem oder mehreren Schnitten geschliffen wird.

Die Rillen 10 in der Oberfläche können auch mit dieser Schleifmaschine erzeugt werden, wenn diese in einer Anordnung für den entsprechenden Zweck vorgesehen wird; in diesem Fall besteht die aus Rillen bestehende Schraffur der peripheren Oberfläche aus parallelen Umfangsringen 10 mit einem Abstand untereinander zwischen 0,5 mm und 3 mm und bevorzugt zwischen 1 mm und 2 mm (Figur 2B).

Wie man aus der Figur 2C erkennen kann haben sowohl die Rillen 9 als auch die Rillen 10 eine sehr geringe Tiefe von weniger als 0,2 mm, und bevorzugt von weniger als 0,1 mm um ihre Aufgabe zu erfüllen, nämlich die Entfernung von fremden Teilchen, wie Läuse und/oder Nissen, die an den zu reinigenden Haaren anhaften, ohne dabei gleichzeitig die Haare zu beschädigen.

Danach wird jeder Zahn 3, der mit einer Rille 9 oder mit den Rillen 10 in seiner peripheren Oberfläche versehen ist, zu einer Vibrationspoliermaschine gebracht, für die endgültige Abrundung der konischen Spitze 11 des Anwendungsende des Zahns 3, wie sie in den Figuren 2A und 2D zu sehen ist. Die Länge des Konus beträgt ca. das 1- bis 4-fache des Durchmessers des zylindrischen Elements und entspricht bevorzugt dem 3-fachen des Durchmessers des zylindrischen Elements. Der Konus endet in einer Halbkugel mit einem Durchmesser von ca. 0,6 mm (Figur 2D).

Die auf diese Weise vorbereiteten Zähne 3 werden auf der Innenseite der ersten Schale 7 parallel nebeneinander angeordnet mit einem Abstand zwischen zwei benachbarten Zähnen 3 von zwischen 50µm und 100µm aber weniger als 100µm und bevorzugt von ca. 90µm. Die Zähne 3 werden so angeordnet, daß außerhalb des Griffs 2 eine Nutzlänge, die zum Kämmen und Reinigen dient, zwischen 30 mm und 50 mm, und bevorzugt von ca. 35 mm gelassen wird, gemessen von dem geraden Rand 12 (Figur 1A) des Griffs 2, und haben eine Gesamtlänge zwischen 40 mm und 80 mm und bevorzugt von ca. 55 mm, um damit dem Kamm 1 eine große Wirksamkeit in Bezug auf jeden beliebigen mit demselben zu reinigenden Haartyp zu verleihen.

Beim Anordnen der Zähne 3 auf der ersten Schale 7 werden dieselben leicht versetzt gegenüber dem jeweils benachbarten Zahn 3 positioniert, so daß ihre Anwendungsenden als Gesamtheit einen gekrümmten Rand 6 bilden (Figur 1A).

Die Befestigung der Zähne 3 auf der ersten Schale 7 kann mit Hilfe einer Schweißung mit Zinn oder durch eine Laserschweißung eines Zahnes 3 nach dem anderen erfolgen, wobei mindestens 6 mm zwischen dem geraden Rand 12 des Griffs 2 und dem Beginn der Schweißung freigelassen werden müssen.

Im Falle, der Verschweißung der Zähne 3 auf der ersten Schale 7 mit dem Laser, kann der Laserstrahl ohne Unterschied sowohl von der Außenseite der ersten Schale 7, als auch von der Seite der Zähne 3 angewandt werden.

Wenn der Laser von der Außenseite der ersten Schale 7 angewandt wird, wird jeder Zahn zuerst an zwei 6 mm von einander entfernten Punkten 5 und mit einem Abstand von mehr als 6 mm vom geraden Rand 12 der ersten Schale 7 verschweißt, in Übereinstimmung mit den jeweiligen Längsachsen der Zähne 3 um dieselben richtig zu positionieren und um zu vermeiden, daß sie wieder aus ihrer korrekten Position verschoben werden, und dann wird jeder Zahn 3 mit einem dritten Punkt 5 in der Mitte zwischen den beiden anderen Schweißpunkten 5 verschweißt.

Wenn der Laser von der Seite der Zähne 3 angewandt wird, werden dieselben mit zwei oder drei Laserschweißpunkten 5 in der Mitte zwischen zwei benachbarten Zähnen 3 in einem äquidistanten Abstand zu den Längsachsen beider Zähne 3 festgeschweißt, aber in jedem Falle in einem Abstand von mehr als 6 mm vom geraden Rand 12 der ersten Schale 7.

Nach dem Verschweißen der Zähne 3 auf der ersten Schale 7, wird die zweite Schale 8 aufgelegt und die runden Ränder der beiden Schalen 7, 8 werden mit einer Vielzahl von Schweißpunkten 5 mit Hilfe des Lasers verschweißt, ohne Unterschied ob die Zähne von der Außenseite der ersten Schale 7 oder von der Seite der Zähne 3 her festgeschweißt wurden.

In dem Fall in dem die Zähne 3 mit Zinn verschweißt werden, wird die Schweißung von der Seite der Zähne 3 angewandt. Danach wird der Griff 2 geschlossen, in dem die zweite Schale 8 unter Druck auf die erste Schale 7 gepreßt wird. In diesem Fall, wird vor der Anwendung des Anpreßdrucks ein in den Figuren nicht dargestelltes Zwischenstück aus Plastik zwischen beiden Schalen 7, 8 eingefügt, welche den Anpreßdruck aufnehmen und tragen muß.

Es versteht sich, daß die vorstehende detaillierte Beschreibung nur zu Zwecken der beispielhaften Anschauung dient, und daß Änderungen und Abweichungen von derselben möglich sind, ohne sich von dem Inhalt der vorliegenden Erfindung zu entfernen.

## Patentansprüche

1. Ein Reinigungskamm (1) mit Zähnen (3), die in ihrer peripheren Oberfläche eine Rauhigkeit aufweisen, welcher aus einem Griff (2) und einer Vielzahl von Zähnen (3) besteht; wobei der Kamm (1) aus einem aseptischen Material hergestellt ist, welches eine Festigkeit hat, die gleich oder größer als die von Stahl ist; in dem der Griff (2) aus einer ersten Schale (7) und aus einer zweiten Schale (8) besteht; in dem jeder Zahn (3) ein Befestigungsende, ein Anwendungsende und eine periphere Oberfläche aufweist; in dem die Befestigungsenden aller Zähne (3) nebeneinander auf der inneren Oberfläche der ersten Schale (7) befestigt sind; in dem die zweite Schale (8) zum Verschließen des Griffs (2) auf der ersten Schale (7) befestigt wird, wodurch die Befestigungsenden aller Zähne eingeschlossen werden; in dem die Anwendungsenden aller Zähne (3) eine geschliffene und abgerundete Spitze (11) aufweisen; **dadurch gekennzeichnet, daß** die periphere Oberfläche jedes Zahnes (3) mit einer Rauhigkeit versehen ist; daß der Abstand zwischen einem Zahn (3) und dem benachbarten Zahn (3) im Bereich zwischen 50µm und 100 µm aber weniger als 100 µm liegt, und daß jeder Zahn (3) eine Gesamtlänge im Bereich zwischen 40 mm bis 80 mm und eine Nutzlänge außerhalb des Griffs (2) zwischen 20 und 60 mm hat.

2. Der Reinigungskamm (1) gemäß dem Anspruch 1, **dadurch gekennzeichnet, daß** der Kamm (1) aus Stahl angefertigt ist.

3. Der Reinigungskamm (1) gemäß dem Anspruch 1 oder dem Anspruch 2, **dadurch gekennzeichnet, daß** die Zähne (3) eine zylindrische Form aufweisen mit einem Durchmesser von mindestens 1 mm.

4. Der Reinigungskamm gemäß einem der Ansprüche (1) bis 3, **dadurch gekennzeichnet, daß** der Abstand zwischen einem Zahn (3) und dem jeweils benachbarten ca. 90 µm beträgt.

5. Der Reinigungskamm gemäß einem der Ansprüche 1 bis 4, **dadurch gekennzeichnet, daß** jeder Zahn (3) eine Gesamtlänge von ca. 55 mm aufweist und eine Nutzlänge, außerhalb des Griffs (2) von nicht weniger als 35 mm.

6. Der Reinigungskamm (1) gemäß einem der Ansprüche 1 bis 5, **dadurch gekennzeichnet, daß** die Vielzahl von Zähnen (3) zwischen 10 und 100 Zähnen umfaßt.

7. Der Reinigungskamm (1) gemäß dem der Anspruch 6, **dadurch gekennzeichnet, daß** die Vielzahl von Zähnen (3) 33 Zähne umfaßt.

8. Der Reinigungskamm (1) gemäß einem der Ansprüche 1 bis 7, **dadurch gekennzeichnet, daß** jeder Zahn (3) leicht versetzt in Bezug auf seine Nachbarzähne (3) angeordnet ist, so daß die Gesamtheit der Anwendungsenden einen gekrümmten Rand (6) bildet.

9. Der Reinigungskamm (1) gemäß einem der Ansprüche 1 bis 8, **dadurch gekennzeichnet, daß** die Rauhigkeit der peripheren Oberfläche jedes Zahns (3) aus einer jeweiligen schraubenförmigen Rille (9) mit einem Gewindegang von weniger als 4 mm besteht.

10. Der Reinigungskamm (1) gemäß dem der Anspruch 9, **dadurch gekennzeichnet, daß** die schraubenförmige Rille (9) einen Gewindegang von weniger als 2 mm hat.

11. Der Reinigungskamm (1) gemäß einem der Ansprüche 1 bis 8, **dadurch gekennzeichnet, daß** die Rauhigkeit der peripheren Oberfläche jedes Zahns (3) aus mehreren parallelen ringförmigen Rillen (10) mit einem Abstand untereinander zwischen 0,5 mm und 3 mm besteht.

12. Der Reinigungskamm (1) gemäß dem Anspruch 11, **dadurch gekennzeichnet, daß** die parallelen ringförmigen Rillen (10) einen Abstand untereinander zwischen 1,0 mm und 2,0 mm haben.

13. Der Reinigungskamm (1) gemäß einem der Ansprüche 9 bis 12, **dadurch gekennzeichnet, daß** die Rillen (9, 10) eine Tiefe von weniger als 0,2 mm haben.

14. Der Reinigungskamm (1) gemäß dem Anspruch 13, **dadurch gekennzeichnet, daß** die Rillen (9, 10)eine Tiefe von weniger als 0,1 mm haben.

15. Der Reinigungskamm (1) gemäß einem der Ansprüche 1 bis 14, **dadurch gekennzeichnet, daß** er außerdem ein Zwischenteil aus Plastik umfaßt, welches zwischen der ersten Schale (7) und der zweiten Schale (8) angeordnet ist und in der Lage ist den Anpreßdruck beim Verschließen des Griffs (2) durch Anpressen aufzunehmen und zu tragen.

16. Eine Methode zur Herstellung eines Reinigungskammes (1) mit hoher mechanischer Festigkeit, **dadurch gekennzeichnet daß** besagter Kamm (1) aus einem aseptischen Material hergestellt ist, welches eine Festigkeit hat, die gleich oder größer als die von Stahl ist, worin die Methode die folgenden Schritte beinhaltet:
eine Vielzahl von Zähnen (1) mit einer Gesamtlänge zwischen 40 und 80 mm zur Verfügung stellen;
eine Rauhigkeit in der peripheren Oberfläche jedes Zahnes (3) erzeugen;
die griffentfernte Spitze (11) an dem Anwendungsende jedes Zahnes (3) anspitzen und abrunden;
die griffnahen Befestigungsenden der Zähne (3) nebeneinander auf der Innenseite einer ersten Schale (7) eines Griffs (2) in einem Abstand zwischen 50 µm und 100 µm aber weniger als 100 µm voneinander getrennt derart positionieren, daß die Nutzlänge der Zähne (3) außerhalb des Griffs (2) zwischen 30 mm und 50 mm beträgt;
die Zähne (3) derart leicht gegeneinander verschoben anordnen, so daß die Gesamtheit der Anwendungsenden einen gekrümmten Rand (6) bilden;
jeden einzelnen der Zähne (3) beständig auf besagter erster Schale (7) befestigen um so dem Kamm (1) eine hohe mechanische Festigkeit zu geben;
und den Griff (2) mit einer zweiten Schale (8) verschließen.

17. Die Methode gemäß dem Anspruch 16, **dadurch gekennzeichnet, daß** der Schritt der Erzeugung einer Rauhigkeit die folgenden Schritte enthält:
ein Schneidwerkzeug auf einer Werkzeugträgervorrichtung anbringen, wobei die Vorrichtung eine Feder aufweist, um einen gleichförmigen Druck auf einen Richtrollengang in einer Drahtricht- und schneidemaschine auszuüben;
einen Zahn (3) der Vielzahl von Zähnen (3) in die Drahtrichtmaschine einbringen; und den besagten Richtrollengang gemeinsam mit besagtem Schneidegerät um den Zahn (3) drehen, wobei aus diesem ein Span in Form einer Rille (9) herausgetrennt wird; und
gleichzeitig den Zahn (3) durch den Richtrollengang vorwärtsbewegen, wodurch schließlich die Rille (9) eine Schraubenform in der peripheren Oberfläche des Zahnes (3) bildet;
und die Schritte, ausgehend von dem Einbringen eines Zahnes (3) in die Drahtrichtmaschine, für jeden der Vielzahl von Zähnen (3) wiederholen.

18. Die Methode gemäß dem Anspruch 17, **dadurch gekennzeichnet, daß** der Gewindegang der Schraubenrille (9) kleiner als 2 mm ist.

19. Die Methode gemäß dem Anspruch 16, **dadurch gekennzeichnet, daß** die Erzeugung einer Rauhigkeit die folgenden Schritte umfaßt:
einen Zahn (3) in eine Schleifmaschine einbringen; und
parallele ringförmige Rillen (10) in die periphere Oberfläche dieses Zahnes (3) schneiden.

20. Die Methode gemäß dem Anspruch 19, **dadurch gekennzeichnet, daß** der Abstand zwischen den Rillen (10) zwischen 0,5 und 3 mm beträgt.

21. Die Methode gemäß einem der Patentansprüche 17 bis 20, **dadurch gekennzeichnet, daß** die Rillen (9, 10) eine Tiefe von weniger als 0,2 mm haben.

22. Die Methode gemäß dem Anspruch 21, **dadurch gekennzeichnet, daß** die Rillen (9, 10) eine Tiefe von weniger als 0,1 mm haben.

23. Die Methode gemäß einem der Patentansprüche 16 bis 22, **dadurch gekennzeichnet, daß** der Schritt der Befestigung der Zähne (3) durch Laserverschweißung ausgeführt wird, und wobei das Verschließen des Griffes (2) den Schritt umfaßt, besagte zweite Schale (8) auf besagter erster Schale (7) mit Hilfe des Lasers zu verschweißen.

24. Die Methode gemäß dem Anspruch 23, **dadurch gekennzeichnet, daß** besagte Laserschweißung durchgeführt wird, in dem der Laserstrahl von der Außenseite der ersten Schale (7) her angewandt wird, und die Schritte umfaßt: jeden Zahn (3) zu positionieren und mit einem oder zwei ersten Laserschweißpunkt (5) in der jeweiligen Position des Zahns (3) anzuheften; und jeden Zahn (3) mit mindestens einem weiteren Laserschweißpunkt (5) beständig zu befestigen, wobei der weitere Laserschweißpunkt (5) in genügender Entfernung von dem einen ersten oder zwischen den zwei ersten Laserschweißpunkten (5) angebracht wird.

25. Die Methode gemäß dem Anspruch 23, **dadurch gekennzeichnet, daß** besagte Laserschweißung durchgeführt wird, in dem der Laserstrahl von der Seite der Zähne (3) her angewandt wird und ein oder mehrere Laserschweißpunkte (5), in Positionen zwischen dem zu befestigenden Zahn (3) und dem benachbarten Zahn (3) angebracht werden.

26. Die Methode gemäß einem der Patentansprüche 16 bis 22, **dadurch gekennzeichnet, daß** die Befestigung der Zähne (3) auf der ersten Schale (7) durch das Verschweißen mit Zinn ausgeführt wird, wobei die Schweißung von der Seite der Zähne (3) her angewandt wird; und wobei das Verschließen des Griffes (2) die Schritte umfaßt: ein Zwischenstück aus Plastik zwischen der ersten (7) und der zweiten (8) Schale einzufügen; und unter Druck die zweite Schale (8) auf der ersten Schale (7) anzupressen, wobei das Zwischenstück aus Plastik den Zweck hat den Anpreßdruck zu tragen.

## Claims

1. A cleaning comb (1) with teeth (3) having a ruggedness on their peripheral surface, which comprises a handle (2) and a plurality of teeth (3); wherein said comb (1) is made of an aseptic material having a mechanical strength equal or greater than steel; said handle (2) comprising a first plate (7) and a second plate (8); each tooth (3) comprising a fastening end, a leading end and a peripheral surface; the fastening end of the teeth (3) being attached side by side on the inner surface of the first plate (7); the second plate (8) being attached to the first plate (7) to close the handle (2) thereby enclosing the fastening ends of all teeth (3); and the leading end of each tooth (3) having a pointed and rounded tip (11) **characterized by** said peripheral surface of each tooth (3) being provided with a ruggedness; the distance between any tooth (3) and its neighboring tooth (3) being in the range of 50 µm to 100 µm, but less than 100 µm; and each tooth (3) having a total length in the range of 40 to 80 mm and a useful length, outside the handle (2) in the range of 20 to 60 mm.

2. The cleaning comb (1) according to claim 1, **characterized in that** the comb (1) is made of steel.

3. The cleaning comb (1) according to claim 1 or claim 2, **characterized in that** each tooth (3) has a cylindrical shape with a diameter of at least 1 mm.

4. The cleaning comb (1) according to any of claims 1 through 3, **characterized in that** the distance between any tooth (3) and the respective neighboring one is of about 90 µm.

5. The cleaning comb (1) according to any of claims 1 through 4, **characterized in that** each tooth (3) has a total length in the range of about 55 mm and a useful length, outside the handle (2), not less than 35 mm.

6. The cleaning comb (1) according to any of claims 1 through 5, **characterized in that** the plurality of teeth (3) comprises between 10 and 100 teeth (3).

7. The cleaning comb (1) according to claim 6, **characterized in that** the plurality of teeth (3) comprises 33 teeth (3).

8. The cleaning comb (1) according to any of claims 1 through 7, **characterized in that** each tooth (3) is slightly offset from the respective neighboring tooth such that the leading ends altogether form a curved edge (6).

9. The cleaning comb (1) according to any of claims 1 through 8, **characterized in that** the ruggedness on the peripheral surface of each tooth (3) is a helical groove (9) with a pitch of under 4 mm.

10. The cleaning comb (1) according to claim 9, **characterized in that** said helix (9) has a pitch of less than 2 mm.

11. The cleaning comb (1) according to any of claims 1 through 8, **characterized in that** the ruggedness on the peripheral surface of each tooth (3) consists of parallel circumferential grooves (10) being spaced at a distance of 0,5 to 3 mm from each other.

12. The cleaning comb (1) according to claim 11, **characterized in that** said parallel circumferential grooves (10) are spaced at a distance of 1 to 2 mm from each other.

13. The cleaning comb (1) according to any of claims 9 through 12, **characterized in that** the depth of said grooves (9, 10) is less than 0,2 mm.

14. The cleaning comb (1) according to claim 13, **characterized in that** the depth of said grooves (9, 10) is less than 0,1 mm.

15. The cleaning comb (1) according to any of claims 1 through 14, **characterized in that** the comb (1) further comprises a plastic interposed member disposed between the first plate (7) and the second plate (8), and which is capable of absorbing and supporting the pressure on press closing the handle (2).

16. A method of manufacturing a high mechanical strength cleaning comb (1), **characterized by** said comb (1) being made of an aseptic material having a mechanical strength equal or greater than steel; said method comprising the steps of
providing a plurality of teeth (3), having a total length of 40 to 80 mm;
creating ruggedness on the peripheral surface of each tooth (3);
sharpening the distal tip (11) of the leading end of each tooth (3) and rounding it;
placing the fastening ends of the teeth (3) side by side at a distance of 50 µm to 100 µm, but less than 100 µm, from each other on the inner side of the first handle plate (7), so that they have a useful length, outside the handle (2), of 30 mm to 50 mm,
arranging the teeth (3) slightly offset such that their leading ends form a curved edge (6);
firmly attaching the teeth (3) one by one onto said first plate (7), thus giving the comb (1) a high mechanical strength; and
sealing the handle (2) with the second plate (8).

17. The method according to claim 18, **characterized in that** the step of creating a ruggedness includes the steps of
placing a cutting tool on a tool-holder device having a spring for yielding a constant pressure, on a straightening roller train of a wire straightener-cutter;
placing one tooth (3) of the plurality of teeth (3) in the wire. straightener, and turning said straightening roller train together with said cutting tool about the tooth (3), thus cutting a chip in the shape of a groove (9) and, at the same time:
advancing the tooth (3) through the straightening roller train, so that the groove (9) finally takes a helical shape on the peripheral surface of the tooth (3); and
repeating said steps beginning with the placing step for each of the plurality of teeth (3).

18. The method according to claim 19, **characterized in that** the pitch of the helix (9) is less than 2 mm.

19. The method according to claim 16, **characterized by** the step of creating a ruggedness including the steps of
placing a tooth (3) inside a grinder;
cutting circumferential parallel grooves (10) on the peripheral surface of the tooth (3).

20. The method according to claim 19, **characterized by** the distance between the grooves (10) being between 0,5 and 3 mm.

21. The method according to any of claims 17 through 20, **characterized in that** the grooves (9, 10) have a depth less than 0,2 mm.

22. The method according to claim 21, **characterized in that** the grooves (9, 10) have a depth less than 0,1 mm.

23. The method according to any of claims 16 through 22, **characterized in that** the step of firmly attaching the teeth (3) is carried out by laser welding, wherein the step of sealing the handle (2) includes the step of laser welding said second plate (8) onto said first plate (7).

24. The method according to claim 23, **characterized in that** said laser welding is carried out by applying the laser beam from the outer side of the first plate (7), and by including the steps of positioning and fixing each tooth (3) with one or two laser welding points (5) in the site of the tooth (3) to be fixed; and firmly attaching each tooth (3) with at least one further laser welding point (5) in a sufficient distance from the one, or between the two, first laser welding points (5).

25. The method according to claim 23, **characterized in that** said laser welding is carried out by applying the laser beam from the side of the teeth (3) using one or more laser welding points (5) in positions between said tooth (3) to be fixed and the adjacent tooth (3).

26. The method according to any of claims 16 through 22, **characterized in that** the step of attaching the teeth (3) on the first plate (7) is carried out by soft soldering applied from the side of the teeth (3); and the step of sealing the handle (2) includes the steps of interposing a plastic member between the first plate (7) and the second plate (8), and pressing the second plate (8) onto the first plate (7), wherein said interposed plastic member must support the pressure force during press closure of the handle (2).

## Revendications

1. Peigne de nettoyage (1) avec des aiguilles (3) pourvues d'une rugosité sur leur surface périphérique, comprenant une manche (2) et une pluralité d' aiguilles (3); ledit peigne étant réalisé d'un matériau aseptique pourvu d'une résistance mécanique égale ou supérieure à celle de l'acier, ladite manche (2) comprenant une première plaque (7) et une deuxième plaque (8); chacune des aiguilles (3) étant pourvue d'une extrémité de fixation, d'une extrémité d'attaque et d'une surface périphérique, l'extrémité de fixation de chacune des aiguilles (3) étant fixée à la surface intérieure de la première plaque (7) l'une à côté de l'autre; la deuxième plaque (8) étant fixée a la première plaque (7) de façon de pouvoir fermer la manche (2) moyennant quoi les extrémités de fixation de chacune des aiguilles (3) sont enfermées; l'extrémité d'attaque de chacune des aiguilles (3) présentant une pointe (11) aiguisée et arrondie, **caractérisé en ce que** ladite surface périphérique de chacune des aiguilles (3) est pourvue d'une rugosité, la séparation entre une aiguille (3) et l'aiguille voisine (3) se trouvant dans une plage de 50 a 100 µm, néanmoins inférieure à 100 µm; et chaque aiguille (3) possédant une longueur totale dans une plage de 40 à 80 mm et une longueur utile, en dehors de la manche (2), dans une plage de 20 a 60 mm.

2. Le peigne de nettoyage (1) selon la revendication 1, **caractérisé en ce que** le peigne est fait en acier.

3. Le peigne de nettoyage (1) selon la revendication 1 ou 2, **caractérisé en ce que** les aiguilles (3) possèdent une forme cylindrique avec un diamètre d'au moins 1 mm.

4. Le peigne de nettoyage (1) selon l'une quelconque des revendications 1 à 3, **caractérisé en ce que** la séparation entre une aiguille (3) et une autre est d'approximativement 90 µm.

5. Le peigne de nettoyage (1) selon I' une quelconque des revendications 1 à 4, **caractérisé en ce que** chacune des aiguilles (3) a une longueur totale d'approximativement 55 mm et une longueur utile en dehors de la manche, d'au moins 35 mm.

6. Le peigne de nettoyage (1) selon l'une quelconque des revendications 1 à 5, **caractérisé en ce que** la pluralité des aiguilles (3) comprend de 10 à 100 aiguilles (3).

7. Le peigne de nettoyage (1) selon la revendication 5, **caractérisé en ce que** la pluralité des aiguilles comprend 33 aiguilles.

8. Le peigne de nettoyage (1) selon l'une quelconque des revendications 1 à 7, **caractérisé en ce que** les aiguilles (3) se trouvent déplacées par rapport à I' aiguille adjacente (3), l'ensemble des pointes d'attaque formant un bord courbé.

9. Le peigne de nettoyage (1) selon une quelconque des revendications 1 à 8 **caractérisé en ce que** la rugosité de la surface périphérique de chacune des aiguilles (3) est un sillon respectif sous forme d'une hélice (9) dont le pas est inférieur à 4 mm.

10. Le peigne de nettoyage (1) selon la revendication 9, **caractérisé en ce que** le pas de ladite hélice (9) est inférieur à 2 mm.

11. Le peigne de nettoyage (1) selon l'une quelconque des revendications 1 à 8, **caractérisé en ce que** la rugosité de la surface périphérique de chacune des aiguilles (3) est constituée par des sillons parallèles circonférentiels (10) avec une séparation mutuelle de 0,5 à 3 mm.

12. Le peigne de nettoyage (1) selon la revendication 11, **caractérisé en ce que** lesdits sillons parallèles circonférentiels (10) sont séparés entre eux de 1 à 2 mm.

13. Le peigne de nettoyage (1) selon l'une quelconque des revendications 9 à 12, **caractérisé en ce que** les sillons (9, 10) ont une profondeur inférieure à 0,2 mm.

14. Le peigne de nettoyage (1) selon la revendication 13, **caractérisé en ce que** les sillons (9, 10) ont une profondeur inférieure à 0,1 mm.

15. Le peigne de nettoyage (1) selon I 'une quelconque des revendications 1 à 14, **caractérisé en ce qu'**il comprend en plus une pièce intermédiaire disposée entre la première plaque (7) y la deuxième plaque (8) ladite pièce intermédiaire étant capable de supporter la pression de serrement exercée lors de la fermeture de la manche (2) par pression

16. Un procédé pour la manufacture du peigne de nettoyage (1) à haute résistance mécanique, ledit peigne étant fait d'un matériau aseptique dont la résistance est égale ou supérieure à celle de l'acier, ledit procédé comprenant les étapes suivantes:
pourvoir une pluralité d'aiguilles (3) ayant une longueur totale entre 40 y 80 mm;
générer une rugosité sur la surface périphérique de chacune des aiguilles (3);
aiguiser la pointe distale (11) de l'extrémité d' attaque de chacune des aiguilles (3), et l'arrondir;
positionner les extrémités prochaines des aiguilles (3) l'une à côté de l'autre avec une séparation mutuelle de 50 à 100 µm, néanmoins inférieure à 100 µm, sur la face intérieure d'une première plaque (7) d'une manche (2) de façon que la longueur utile des aiguilles (3) dépassant la manche (2) soit de 30 à 50 mm;
arranger les aiguilles (3) légèrement déplacées entre elles de façon que leurs extrémités d'attaque forment un bord courbé;
fixer fermement les aiguilles (3) l'une contre l'autre sur ladite plaque (7), ce qui confère une haute résistance mécanique au peigne (1), et:
fermer la manche (2) moyennant la deuxième (8) plaque.

17. Le procédé selon la revendication 16, **caractérisé en ce que** l'étape de la génération d'une rugosité inclut les étapes suivantes :
placer un outil de coupure sur un dispositif porte-outil, pourvu d'un ressort destiné à exercer une pression constante sur un train de rouleaux redresseurs d'un redresseur et coupeur de fils de fer;
placer une aiguille, faisant partie de la pluralité d'aiguilles (3), dans le redresseur, et faire tourner ledit train de rouleaux redresseurs ensemble avec ledit outil de coupure de façon solidaire autour de l'aiguille (3) en dégageant de la tournure sous forme de sillon (9), de l'aiguille, et, en même temps:
faire avancer l'aiguille à travers du train de rouleaux redresseurs, tout en conformant finalement de cette façon le sillon (9) sous forme d'une hélice sur la surface périphérique de l'aiguille (3); et
répéter les étapes précédentes, à partir de l'étape du placement, pour chaque aiguille (3) de la pluralité des aiguilles (3).

18. Le procédé selon la revendication 27, **caractérisé en ce que** le pas de I' hélice (9) es inférieur à 2 mm.

19. Le procédé selon la revendication 16, **caractérisé en ce que** I' étape de la génération d'une rugosité inclut les étapes suivantes:
placer l'aiguille (3) dans un redresseur;
couper des sillons parallèles circonférentiels (10) sur la surface périphérique de l'aiguille (3).

20. Le procédé selon la revendication 19, **caractérise en ce que** la séparation entre les sillons (10) est de 0,5 à 3 mm.

21. Le procédé selon l'une quelconque des revendications 17 à 20, **caractérisé en ce que** les sillons (9, 10) ont une profondeur inférieure à 0,2 mm.

22. Le procédé selon la revendication 21, **caractérise en ce que** les sillons (9, 10) ont une profondeur inférieure à 0,1 mm.

23. Le procédé selon l'une quelconque des revendications 16 à 22, caractérise en ce l'étape de la fixation des aiguilles (3) es réalisée moyennant soudure laser, et en ce que ladite étape de fermeture de la manche (2) inclut l'étape de la soudure laser de ladite deuxième plaque (8) à ladite première plaque (7).

24. Le procédé selon la revendication 23, **caractérise en ce que** ladite soudure laser est réalisée en attaquant avec le rayon laser du côté extérieur de la première plaque (7), et inclut les étapes de la fixation de chacune des aiguilles (3) sur sa place respective moyennant un ou deux points de soudure laser (5); et tenir fermement chacune des aiguilles (3) moyennant tout au moins un point de soudure laser (5) additionnel a une distance suffisante du premier point de soudure laser (5) ou entre les deux premiers points de soudure laser (5).

25. Le procédé selon la revendication 23, **caractérise en ce que** ladite soudure laser est réalisée en attaquant avec le rayon laser du côté des aiguilles (3) en appliquant un ou deux points de soudure laser (5) entre l'aiguille (3) à fixer et l'aiguille adjacente (3).

26. Le procédé selon l'une quelconque des revendications 16 à 22, **caractérisé en ce que** l'étape de la fixation est réalisé moyennant soudure étain en attaquant du côté des aiguilles (3); et **en ce que** ladite étape de fermer la manche (2) inclut les étapes de placer une pièce intermédiaire de matériau plastique entre la première plaque (7) et la deuxième (8), et serrer la deuxième plaque (8) contre la première plaque (7), et ladite pièce intermédiaire de matériau plastique supportant la pression de serrage.
